(19) Europäisches Patentamt
European Patent Office
Office européen des brevets

(11) Publication number: 0 375 414 B1

(12) EUROPEAN PATENT SPECIFICATION

(45) Date of publication of patent specification: 15.06.94

(51) Int. Cl.5: C07D 403/04, C07D 239/12, C07D 233/48, A01N 43/50, A01N 43/54

(21) Application number: 89313421.3

(22) Date of filing: 21.12.89

# (54) Substituted guanidine and amidine compounds, their production and fungicidal use.

(30) Priority: 22.12.88 GB 8829935

(43) Date of publication of application:
27.06.90 Bulletin 90/26

(45) Publication of the grant of the patent:
15.06.94 Bulletin 94/24

(84) Designated Contracting States:
AT BE CH DE ES FR GB IT LI NL SE

(56) References cited:
US-A- 2 899 426

(73) Proprietor: DowElanco
9002 Purdue Road
Indianapolis, Indiana 46268-1189(US)

(72) Inventor: Liebeschuetz, John W.
34 Grove Street
Wantage Oxfordshire OX12 7AA(GB)
Inventor: Jung, Michel J.
22, Via Narconi
I-21049 Tradate(IT)

(74) Representative: Raynor, John et al
W.H. Beck, Greener & Co
7 Stone Buildings
Lincoln's Inn
London WC2A 3SZ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 375 414 B1

## Description

This invention concerns novel chemical compounds containing a guanidine or an amidine grouping, their preparation, compositions containing them, and their method of use as fungicides.

Many substituted guanidines are known to have antifungal and antibacterial activity. A detailed review of the antifungal activity of such compounds is found in international pest control, November/December 1986, pp 148-155 (H.R. Hudson, I.A.O. Ojo, and M. Pianka).

Fungicidally effective amidine-type compounds are also disclosed in Czechoslovakian Patent No. 197976. Fungicidally active guanidine-containing compounds are also known, for example in U.S. Patent 2,988,478.

Suprisingly, the present invention provides novel chemical compounds containing a guanidine or an amidine grouping, which compounds serve as active ingreidents in fungicidal compositions useful in killing and controlling various fungal organisms which infest plants. Many of these compounds not only kill and control fungal organisms, but, in contrast to the prior art compounds, also provide systemic protection and certain forms of the compounds which are very water soluble allow for easier formulating responses.

Specifically, the compounds of the present invention correspond to the general formula

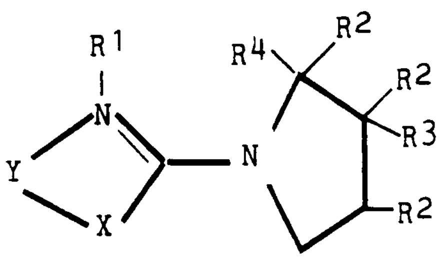

I

or an agriculturally acceptable salt thereof,
wherein:

$R^1$ is hydrogen or $C_1$-$C_3$ alkyl, with the proviso that if the compound is not in the salt form, then $R^1$ is absent;

each $R^2$ independently is hydrogen or $C_1$-$C_4$ alkyl;

$R^3$ is a $C_4$-$C_{20}$ alkyl group, a $C_4$-$C_{20}$ alkenyl group, a phenyl group, a phenyl-$C_1$-$C_3$ alkylene group or a phenyl-$C_2$-$C_3$ alkenylene group wherein the phenyl ring can be substituted with from 1 to 5 halogen atoms, $C_1$-$C_6$ alkyl, $C_1$-$C_6$ alkoxy groups, trihalomethyl groups, phenyl groups or phenoxy groups;

$R^4$ is hydrogen or $C_1$-$C_4$ alkyl;

Y is a group of the formula $-(CR^7R^7)_n-$,
wherein n is 2, 3 or 4, each $R^7$ independently is hydrogen or $C_1$-$C_4$ alkyl, $C_1$-$C_4$ hydroxyalkyl, $C_2$-$C_4$ alkoxyalkyl, or a group of the formula $-(CH_2)_q$-O(CO)-$R^8$,
wherein q is 1, 2 or 3, and $R^8$ is methyl or ethyl; and

X is a group of the formula $-CR^7R^7$,
wherein each $R^7$ independently is as defined above, or a group of the formula $-N(R^9)-$,
wherein $R^9$ is H, or $C_1$-$C_3$ alkyl.

It will of course be appreciated that although the compound of the Formula I is illustrated as containing a simple double bond, in practice considerable delocalization of the double bond structure will take place over the two or three nitrogen atoms of the compound. The compounds of Formula I may contain two or three basic nitrogen atoms, and thus can exist either in the form of the free base or a salt, for example a quaternary salt or an acid addition salt. The compound can thus exist in tautomeric forms, all of which are within the scope of the present invention.

In the compounds of the Formula I, $R^1$ is preferably hydrogen, methyl, ethyl or is absent, more preferably hydrogen or it is absent, and $R^2$ is preferably hydrogen or methyl, more preferably hydrogen, and $R^4$ is preferably methyl.

Y is preferably a group of the formula $-(CR^7R^7)_3-$,wherein $R^7$ is as defined above (preferably hydrogen or methyl), more preferably a group of the formula $-(CH_2)_3-$or $-CH(CH_3)CH_2CH(CH_3)-$.

The group represented by X in Formula I is an optionally substituted carbon atom (in which case the compound is of the amidine type), or an optionally substituted nitrogen atom (in which case the compound is of the guanidine type). Preferably, the $R^7$ groups are each independently hydrogen or methyl, more

preferably hydrogen and the group $R^9$ is preferably hydrogen or methyl, more preferably hydrogen.

The $R^3$ group is preferably a phenyl group optionally substituted with from 1 to 5 halogen atoms, $C_1$-$C_6$ alkyl or $C_1$-$C_6$ alkoxy groups, trihalomethyl, phenyl or phenoxy groups, more preferably a phenyl group substituted in the 4-ring position with a $C_3$-$C_6$ alkyl group.

The ring containing the guanidine/amidine group is preferably six membered, i.e. n in the definition of Y is preferably 3.

The terms alkyl, alkylene, alkenylene and the like, as used herein are intended to include within their scope both straight and branched groups, and the terms alkenyl, alkenylene and the like are intended to cover groups containing one or more than one double bonds. The term halogen and halo include chlorine, bromine, fluorine and iodine.

The invention includes within its scope salts, particularly agriculturally acceptable salts of the compounds of Formula I, for example, the chloride, bromide, acetate, stearate, benzoate and dodecylbenzenesulphonate, with the chloride being preferred.

The compounds of Formula I and their salts may be prepared by:

(A) reacting a compound of the Formula

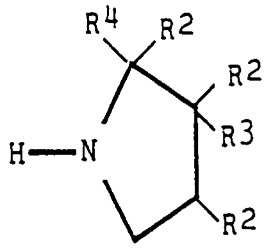

II

wherein $R^2$, $R^3$, and $R^4$ are each as defined as above, with a compound of the formula

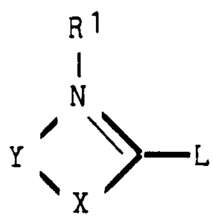

III

wherein $R^1$ is defined as above, or a halide salt thereof, wherein the term "halide" represents bromo, chloro or iodo; and L is a suitable leaving group, such as for example, an alkoxy, an alkylthio (preferably methylthio or ethylthio), phenylthio or sulfonic acid ($SO_3H$) group. The reaction may be carried out at a temperature of from 50 to 200°C, neat (in the absence of solvent) or in a suitable organic solvent, for example an alkanol such as ethanol, n-propanol, isopropanol, n-butanol, isobutanol, n-pentanol or n-hexanol.

This process may be used for example for the preparation of the amidine-type compounds of Formula I (i.e. those in which X is a group of the formula $-CR^7R^7-$), in which case L is preferably an alkoxy group and $R^1$ is absent. Additionally, this process may also be used for the preparation of guanidine type compounds of Formula I (i.e. those in which X is a group of the formula $-N(R^9)-$ in which case, L is preferably an alkylthio group, and the compound of Formula III is used in the form of a halide salt thereof.

The guanidine-type compounds of Formula I [i.e. those in which X is a group of the formula $-N(R^9)-$] which correspond to the formula

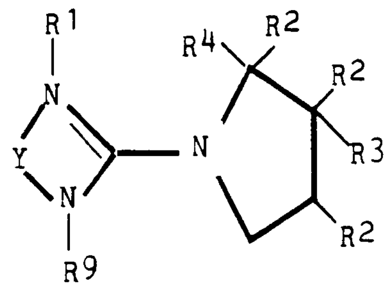

Ia

wherein:

$R^2$, $R^3$, $R^4$ and Y are defined as above,

may also be prepared by:

(B) reducing a compound of formula

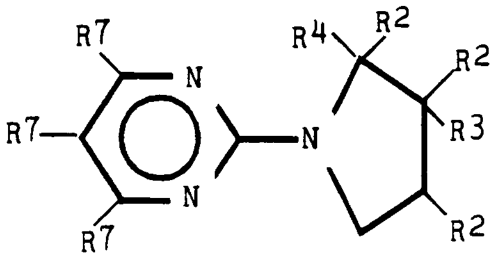

V

wherein:

$R^2$, $R^3$, $R^4$ and $R^7$ are defined as above,

preferably utilizing hydrogen and a conventional metallic hydrogenation catalyst, such as palladium, platinum, rhodium or ruthenium. The reaction may be carried out at any suitable temperature and pressure, preferably room temperature and atmospheric pressure, in a polar solvent, such as for example, water, methanol, ethanol or mixtures thereof and in the presence of an acid such as hydrochloric or acetic acid.

The compound of Formula V in may be prepared by reacting a compound of the formula

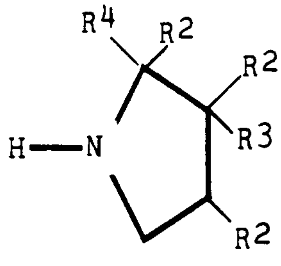

II

wherein:

$R^2$, $R^3$ and $R^4$ are as defined as above,

with a compound of the formula

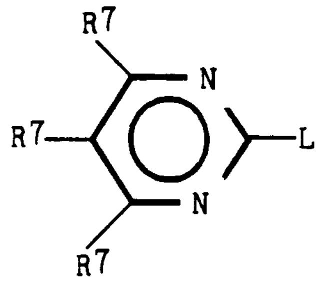

IV

wherein:

$R^7$ is as defined as above, and L is a suitable leaving group.

The reaction of the compound of Formula II with the compound of Formula IV may be carried out in a polar solvent for example, an alkanol such as ethanol, n-propanol, n-butanol, n-pentanol or n-hexanol or a solvent such as dimethylforamide or dimethylsulfoxide in the presence of a base conventionally used as an acid acceptor, such bases include pyridine and trialkylamines, such as triethylamine and ethyldiisopropylamine, at a temperature of from 50 to 200 ° C.

Compounds of Formula II may in turn be prepared by the internal condensation of a compound of the formula

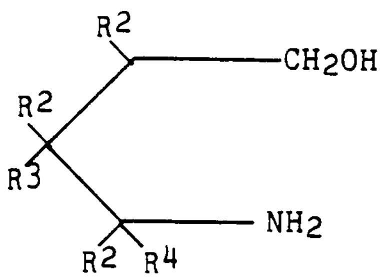

VI

wherein:

$R^2$, $R^3$ and $R^4$ are defined as above,

preferably in the presence of triphenylphosphine and diethyl azadicarboxylate. The internal condensation may be carried out in an organic solvent, for example an ether such as tetrahydrofuran (THF), at a temperature of from 0 to 150 ° C.

Compounds of the Formula VI may be prepared by the reaction of a compound of the formula

$R^4R^2CH \cdot NO_2$

with a compound of the formula

$R^2R^3C = CR^2CO_2C_2H_5$

preferably in the presence of a mild base, such as tetrabutyl ammonium hydroxide or tetrabutyl ammonium fluoride, at a temperature of from 25 to 150 ° C, followed by reduction of the nitro and ester groups, for example using a metal hydride such as $LiAlH_4$. The reduction may be carried out at a temperature of from minus 80 to 100 ° C in an inert solvent, for example an ether.

The compound of formula

$R^2R^3C = CR^2CO_2C_2H_5$

may in turn be prepared by reaction of a compound of the formula

$C(O)R_2R_3$

with a compound of the formula

$(C_2H_5O)_2PCHR^2CO_2C_2H_5$

in presence of a base, such as sodium hydride. The reaction may be carried out in a solvent, such as diethyl ether or tetrahydrofuran (THF), at temperatures of from 0 to 50 ° C.

A number of preferred embodiments of the invention are illustrated in the following Examples. However, these examples are for illustration only and not to be construed as limiting the scope of the present invention.

Example 1: *Cis* N-(tetrahydropyrimidin-2-yl)-2-methyl-3-(4-*t*-butylphenyl)pyrrolidinium acetate).

(a) Preparation of ethyl 3-(4-*t*-butylphenyl) propenoate.

2.42 Grams (g) (0.06 mol) of sodium hydride (60 percent in mineral oil) was washed with hexane under nitrogen. To the sodium hydride was added 30 mL of tetrahydrofuran and the mixture cooled to 0 ° C. To the mixture was added dropwise, with stirring, 13.8 g (0.06 mol) of triethylphosphonoacetate in 50 mL of THF. Half an hour after completion of the addition, 10 g (0.06 mol) of 4-*t*-butyl-benzaldehyde in 40 mL THF was added dropwise. The mixture was allowed to warm to room temperature, the solvent was evaporated and the residue was taken up in ethylacetate. The resulting solution was washed four times with water, dried, filtered and the solvent removed under reduced pressure to afford 12 g (81 percent of theoretical) of the crude product in the form of an oil. The NMR and IR spectra of the product were consistent with the expected structure.

(b) Preparation of Ethyl 4-nitro-3-(4-t-butylphenyl)pentanoate

To 6 g (0.026 mol) of the compound prepared in (a) above, 35 mL (0.49 mol) of nitroethane and 6 mL of a 40 percent solution of tetrabutylammonium hydroxide in methanol were added, mixed and refluxed together under nitrogen for 3 hours. The resulting mixture was poured into 85 ml of a 10 percent aqueous ammonium chloride solution and the resultion solution was thoroughly extracted with ethylacetate. The organic solution was washed three times with 10 percent $NH_4Cl$, dried, filtered and the solvent evaporated off to afford 4.2 g (53 percent of theoretical) of the crude product in the form of an oil. Elemental analysis indicated the following:

| | percent | | |
|---|---|---|---|
| | C | H | N |
| calculated | 66.4 | 8.20 | 4.6 |
| found | 66.4 | 8.30 | 4.6. |

The NMR and IR spectra of the product were consistent with the expected structure.

(c) Preparation of 3-(4-t-butylphenyl)-4-aminopentan-1-ol.

To 6.5 g (0.021 mol) of the nitroester prepared as in (b) above was added dropwise 5 g (0.18 mol) of lithium aluminum hydride suspended in 200 mL of anhydrous diethyl ether, under nitrogen with stirring. The mixture was then refluxed for ten hours. A 50 percent $H_2O$/THF solution was added dropwise and slowly with vigorous stirring until effervescence ceased. To the mixture was added 1N sodium hydroxide solution dropwise until the slurry coagulated to a coarse sandy consistency. The mixture was filtered, and the solid washed with diethyl ether. The organic fractions were combined, washed with water, dried, filtered and the solvent evaporated at reduced pressure to afford 4 g (80 percent of theoretical) of the crude product in the form of a gum. The NMR and IR spectra of the product were consistent with the expected structure, and indicated a mixture of the two possible diastereoisomers in a ratio of 2:1.

(d) Preparation of 3-(4-*t*-butylphenyl)-2-methylpyrrolidine.

To 100 mL THF was added 4.2 g (0.018 mol) of the aminoalcohol prepared in (c), 4.69 g (0.018 mol) of triphenylphosphine and 3.1 g (0.018 mol) of diethyl azodicarboxylate. To the solution was added 2 drops of glacial acetic acid and the solution refluxed for 2 hours. During this time the initially red solution became yellow. On cooling, hexane was added dropwise until precipitation started. The mixture was then cooled in the freezer and the crystalline precipitate filtered off. The solvent was removed under reduced pressure and the residue taken up in chloroform and washed with water. The organic layer was dried with $MgSO_4$, filtered, the solvent evaporated under reduced pressure and the residue vacuum distilled [130 °C at 2 mm Hg (266 Pa)] to afford 3.0 g (77 percent of theoretical) of the crude product in the form of a mobile oil. The NMR and IR spectra of the product were consistent with the expected structure and indicated a mixture of the two possible diastereoisomers were present in a ratio of 3:1.

(e) Preparation of *cis* and *trans* 1-(pyrimidin-2-yl)-2-methyl-3-(4-*t*-butylphenyl)pyrrolidine.

To 1.4 g (0.0065 mol) of the substituted pyrrolidine prepared in (d), 0.74 (0.0065 mol) g of chloropyrimidine and 0.83 g (0.0065 mol) of diisopropylethylamine were added, mixed together in 40 mL pentanol, and refluxed under nitrogen for six hours. The pentanol was distilled off under reduced pressure. The residue was taken up in dichloromethane and washed three times with water. The solution was dried over $MgSO_4$ and filtered. The solvent was evaporated under reduced pressure. Column chromatography using a toluene/ethylacetate mixture as the eluent afforded both the *cis* and *trans* isomers stereochemically pure in a 2:1 ratio in a yield of 1.36 g (70 percent of theoretical). Elemental analysis indicated the following:

| | percent | | |
|---|---|---|---|
| | C | H | N |
| calculated | 77.2 | 8.55 | 14.2 |
| found | 76.7 | 8.65 | 13.4. |

(f) Preparation of *cis* N-(tetrahydropyrimidin-2-yl)-2-methyl-3-(4-*t*-butylphenyl)pyrrolidinium acetate.

To 20 mL of ethanol was added 540 mg (0.0018 mol) of the *cis* pyrimidinylamine prepared above in (e), to the solution was added 100 mg of 10 percent Pd/C and 0.5 mL (0.09 mol) of acetic acid. The mixture was hydrogenated at room temperature and pressure with shaking for eight hours until the theoretical quantity of hydrogen had been absorbed. The mixture was passed through Celite™ and the solvent removed by evaporation under reduced pressure. After vacuum drying the residue, 0.5 g (75 percent of theoretical) of a gum was collected. The NMR and IR spectra of the product were consistent with the expected structure.

Example 2: Preparation of *trans* N-(tetrahydropyrimidin-2-yl)-2-methyl-3-(4-*t*-butylphenyl)pyrrolidine acetate.

The *trans* product from step (e) in Example 1 was subjected to the same procedure as in step (f) above to yield 0.25 g (76 percent of theoretical) of the *trans* isomer as the product. The NMR and IR spectra of the product were consistent with the expected structure.

By an analogous process, using one or more of the following: hydrochloric acid in place of acetic acid in step (f); 4,6-dimethyl-2-chloropyrimidine in place of 2-chloro-pyrimidine in step (e); and n-nonanal in place of 4-*t*--butylbenzaldehyde in step (a); the following compounds were prepared:

Example 3: *Cis* N-(tetrahydropyrimidin-2-yl)-2-methyl-3--(4-*t*-butylphenyl)pyrrolidinium chloride, yield of 0.72 g (90 percent of theoretical).

Example 4: *Trans* N-(tetrahydropyrimidin-2-yl)-2-methyl,3-(4-*t*-butylphenyl)pyrrolidinium chloride, yield of 0.24 g (35 percent of theoretical).

Example 5: *Cis* N-(4,6-dimethyltetrahydropyrimidin-2-yl)-2-methyl-3-(4-*t*-butylphenyl)pyrrolidinium chloride, yield of 0.34 g (44 percent of theoretical).

Example 6: *Trans* N-(4,6-dimethyltetrahydropyrimidin-2-yl)-2-methyl-3-(4-*t*-butylphenyl)pyrrolidinium chloride, yield of 0.14 g (36 percent of theoretical).

Example 7: *Cis* N-(tetrahydropyrimidin-2-yl)-2-methyl-3-octyl pyrrolidinium chloride, yield of 0.2 g (55 percent of theoretical).

In all cases, NMR and IR spectra of the product were consistent with the expected structure. Additionally, the products of Examples 1-7 were either obtained in the form of an oil or a gum.

The compounds of Formula I may be used as fungicides, in particular for agricultural use, against a wide range of pathogens, for example *Ascomycetes*, *Eumycetes* and *Fungi Imperfecti*, in a protectant (control and prevent infestation) or eradicant (kill) fashion. The compounds exhibit low phytotoxicity to crops, in particular cereal and broadleaf crops and in accordance with the invention may be applied to the roots, seeds, or foliage of barley and other plants, for the control of various fungi, without damaging their commercial value. In particular the compounds of the present invention effectively control a variety of undesirable fungi which infest useful plant crops. The compounds are particularly effective against *Deuteromycotina* such as *Septoria nodorum* (glume blotch of cereals), *Pyricularia oryzae* (rice-blast), *Botrytis cinerea* (gray mold of grapes) and *Fusarium oxysporum* (various wilt diseases); *Ascomycotina* such as *Pyrenophora teres* (net-blotch) and *Erysiphe graminis* (powdery mildew); and *Basidiomycotina* such as *Puccinio recondita* (leaf rust).

The compounds of Formula I can be applied to the area where infestation can occur such as seeds, roots or foliage of cereals or other plants and will kill or control the growth of various fungi without damaging the commercial value of said plants.

Where the acid addition salt of the compound of Formula I is employed, the improved water solubility (especially of the chloride) also allows the preparation of solutions (in water or an organic solvent) in which, at use rate, the addition salt is completely soluble in the spray dilution.

At least utilizing the preferred embodiments of Formula I, a single application of the compositions containing as the active ingreident at one compound of Formula I can provide a residual control of powdery mildew diseases over an extended period. Also, the compounds of Formula I can be effective in eliminating established barley powdery mildew infestation. Furthermore, many compounds of Formula I have been found to be translocated in plants and, thus, can provide a systemic protection against powdery mildew.

The compounds of Formula I may also find application an non-agricultural fungicides, for example in medicine as antimycotics against organisms such as *Candida albicans*, *Candida spp*, *Trichophyton spp*, *Aspergillus spp*, *Microsporum spp* and *Sporothrix spp*, and also as agents against parasites such as *Leishmania*.

The method includes within its scope a method for the control or prevention of fungal attack, which method comprises applying to the locus of the fungus, or to a locus in which the infestation is to be prevented, (for example applying to cereal grain plants), a fungicidally effective amount of one or more of the compounds of Formula I.

The invention also embraces the employment of a liquid, powder, dust or granular composition containing one or more of the active compounds of Formula I and one or more inert, non-phytotoxic materials, known in the art as agricultural adjuvants and/or carriers, in solid or liquid form. Thus, for example, the active compound(s) of Formula I can be admixed with one or more additives including water or other liquid carriers such as organic solvents, and petroleum distillates, surface active dispersing agents, and finely divided inert solids. In such compositions, the active ingredients are present in a concentration from 2 to 95 percent by weight, preferably 10 to 95 percent by weight, and most advantageously 10 to 75 percent by weight.

The compounds of Formula I can be employed as a composition in the form of a solution, a diluted flowable composition or a wettable powder composition containing 2 to 10,000 ppm, preferably 10 to 600 ppm, of an active ingredient of Formula I. When the carrier contains a surface active agent, from 0.01 to 20

percent by weight of the active ingredient of Formula I is advantageously employed. Depending upon the concentration in the composition, such augmented compositions are adapted to be employed for the control of the undesirable fungi or employed as concentrates and subsequently diluted with additional inert carrier, e.g. water, to produce the ultimate treating compositions. In general, good results can be obtained with liquid compositions containing from 0.0001 to 2.0 percent by weight of the toxicant in the final diluted form. With dusts, good results can usually be obtained with compositions containing from 0.1 to 2.0 percent or more by weight of toxicant.

Where the compositions are to be applied to foliage of plants, it is preferred that the toxicant be present in an amount not to exceed about 0.8 percent in liquid compositions and about 1.0 percent in dusts. In terms of hectare application, good controls of powdery mildews can be obtained when the active ingredients of Formula I are applied to growing plants at a dosage of from 0.004 to 4 kg/hectare. When employed as fungicides for the treatment of seeds or non-living substrates, from 0.1 to 100 gram of active ingredient of Formula I per kilogram of substrate is an effective dose.

In the preparation of dust, or wettable powder compositions, the toxicant products can be compounded with any of the finely divided solids, such as prophyllite, talc, chalk, gypsum, fuller's earth, bentonite, attapulgite, starch, casein, gluten, or the like. In such operations, the finely divided carrier is ground or mixed with the toxicant or wet with a solution of the toxicant in a volatile organic solvent. Also, such compositions when employed as concentrates can be dispersed in water, with or without the aid of dispersing agents, to form spray mixtures. Dust compositions are advantageously employed when treating seeds.

Granular formulations are usually prepared by impregnating a solution of the toxicant in a volatile organic solvent onto a bed of coarsely divided attapulgite, bentonite, diatomite, or the like.

Similarly, the toxicant products can be compounded with a suitable water-immiscible inert organic liquid and a surface active dispersing agent to produce an emulsifiable concentrate which can be further diluted with water and oil to form spray mixtures in the form of oil-in-water emulsions which may optionally contain water miscible organic co-solvents to improve the physical properties of the formulation. In such compositions, the carrier comprises an aqueous emulsion, i.e., a mixture of inert water-immiscible solvent and optional water miscible organic co-solvent, emulsifying agent, and water.

Emulsifiers which an be advantageously employed herein can be readily determined by those skilled in the art and include various nonionic, anionic, cationic and amphoteric emulsifiers, or a blend of two or more emulsifiers. Examples of nonionic emulsifiers useful in preparing the emulsifiable concentrates include the polyalkylene glycol ethers and condensation products of alkyl and aryl phenols, aliphatic alcohols, aliphatic amines or fatty acids with ethylene oxides, propylene oxide or mixtures of ethylene and propylene oxides such as the ethoxylated alkyl phenols and carboxylic esters solubilized with the polyol or polyoxyalkylene. Cationic emulsifiers include quaternary ammonium compounds and fatty amines. Anionic emulsifiers include the oil-soluble salts (e.g., calcium) of alkylaryl sulphonic acids, oil soluble salts or sulphated polyglycol ethers and appropriate salts of phosphated polyglycol ether.

Representative organic liquids which can be employed in preparing the emulsifiable concentrates of Formula I are the aromatic liquids such as xylene; propyl benzene fractions; or mixed naphthalene fractions; mineral oils substituted aromatic organic liquids such as dioctyl phthalate; kerosene; butene; dialkyl amides of various fatty acids, particularly the dimethyl amides of fatty glycols and glycol derivatives such as the n-butyl ether, ethyl ether or methyl ether of diethylene glycol; and the methyl ether of triethylene glycol. Mixtures of two or more organic liquids are also often suitably employed in the preparation of the emulsifiable concentrate. The preferred organic liquids are xylene, and propyl benzene fractions, with xylene being most preferred.

The surface active dispersing agents are usually employed in liquid compositions of this invention and in the amount of from 0.1 to 20 percent by weight of the combined weight of the dispersing agent and active compound of Formula I. The active compositions can also contain other compatible additaments, for example, plant growth regulators and other biologically active compounds used in agriculture.

In particular, these active compositions containing compounds of Formula I may contain adjuvant surfactants to enhance the deposition, wetting and penetration of the composition onto the target crop and organism. These adjuvant surfactants may optionally be employed as a component of the formulation or as a tank mix. The amount of adjuvant surfactant will vary from 0.01 to 1.0 percent v/v based on a spray-volume of water, preferably 0.05 to 0.5 percent. Suitable adjuvant surfactants include ethoxylated nonyl phenols, ethoxylated synthetic or natural alcohols, salts of the esters or sulphosuccinic acids, ethoxylated organosilicones, ethoxylated fatty amines and blends of surfactants with mineral or vegetable oils.

In such embodiments, the compounds of Formula I or compositions containing the compounds of Formula I, can be advantageously employed in combination with one or more additional pesticidal

compounds. Such additional pesticidal compounds may be insecticides, nematocides, miticides, arthropodicides, or bactericides that are compatible with the compounds of Formula I in the medium selected for application and not antagonistic to the activity of the compounds of Formula I. Accordingly, in such embodiments, the pesticidal compound is employed as a supplemental toxicant for the same or for a different pesticidal use or as an additive. The compounds in combination can generally be present in a ratio of from 1:100 to 100:1.

The exact amount of the active material to be applied is dependent not only on the specific active material being applied, but also on the particular action desired, the fungal species to be controlled and the stage of growth thereof as well as the part of the plant to be contacted with the toxic active ingredient. Thus, all of the active ingredients of the present invention containing compounds of Formula I and compositions containing them may not be equally effective at similar concentrations or against the same fungal species.

The compounds of Formula I may be applied in the form of any of the generally used formulation types, for example as solutions, dusts, wettable powders, flowable concentrates, or emulsifiable concentrates.

Fungicidal Activity Test Procedures

The compounds of Examples 1 to 7 were tested in accordance with the following *in vitro* and *in vivo* test methods.

*In vitro* screen method

Test compounds are dissolved in acetone and made up with distilled water to give a solution with a final concentration of compound of 400 ppm and 10 percent acetone. Into sterile petri dishes are pipetted 2 mL aliquots of the latter solutions and 18 mL of agar is then added to each dish using an automatic agar plate pourer to give a final concentration of compound of 40 ppm.

Once the agar is set, discs of pathogens are cut from stock agar plates using a cork borer, and placed (pathogen face down) onto the test agar surface. Pathogens used in the primary screen include:

1. *Alternaria brassicola*
2. *Fusarium oxysporum phaseolicola*
3. *Pyrenophora teres*
4. *Botrytis cinerea*
5. *Pyriculoria oryzae*
6. *Pseudocercosporella herpotrichoides*.

Three pathogens are placed on each plate. Two replicates of each plate are set up and kept in a incubator at 20 ° C.

The pathogens on plate 1 are assessed after 5 days and those on plate 2 after 8 days. The diameters of the fungal colonies are measured after the incubation period and compared to the control measurements. Allowing for the diameter of the original disc, percentage inhibition values are then calculated.

*In Vivo* Screen Method

Compounds are dissolved in acetone or water (as required) and made up with distilled water to give a final concentration of compound of 400 ppm (and 10 percent acetone where acetone is used).

For each compound 3 replicate plants per pathogen at the 1 leaf stage are sprayed to run off. The plants are allowed to dry at room temperature for 24 hours prior to inoculation.

Untreated control plants and plants sprayed with 10 percent acetone-water or water alone (as required) are included for each pathogen.

Method for *Erysiphe graminis Hordei*

Barley cv. Golden Promise is used as the host plant. Seeds are sown 8 per 3 in. (7.5 cm) pot and allowed to germinate and the plants are treated when they are approximately 2 weeks old. Spores are blown onto the test plants from the stock plants, which are then incubated at 20 ° C, relative humidity 70 percent for 7 days. After a week symptoms are recorded. The percent infection is scored from 3 replicate plants and expressed as a percent of the infection on the acetone-water control plants. The percent control is then recorded.

Method for *Puccinio recondita*

Wheat cv. Tonic is used as the host plant. Seeds are sown 1 cm deep in 4 rows per plastic tray. The tray is 60 cm x 30 cm. Seedlings grown to the 1-2 leaf stage are then inoculated. Into 100 mL of distilled water is placed 0.05 g of uredospores with a melting agent. This solution is sprayed onto the seedlings leaving a fine coverage on the leaves. The plants are then incubated with 100 percent relative humidity at 15-20 °C for 4 days.

Percent infection is scored from 3 replicate plants and expressed as a percent of the infection on acetone-water control plants. Percent control is then recorded.

The results are shown in Table 1 and 2 respectively.

TABLE 1

Percent Efficacy of Compounds of Examples 1 to 7 in *In vitro* Biological Evaluation

| Example Number | *Alternaria brassicola* | *Fusarium oxysporum* | *Pyrenophora teres* | *Pseudocercosporella herpotrichoides* | *Botrytis cinerea* | *Pyricularia oryzae* |
|---|---|---|---|---|---|---|
| 1 | 81 | 74 | 100 | NT | 33 | 90 |
| 2 | 61 | 90 | 95 | NT | 49 | 65 |
| 3 | NT | 68 | 100 | 50 | NT | 68 |
| 4 | NT | 86 | 78 | 33 | NT | 47 |
| 5 | NT | 79 | 84 | 67 | NT | 63 |
| 6 | NT | 80 | 75 | 58 | NT | 63 |
| 7 | NT | 84 | 92 | 82 | NT | 85 |

NT - Not Tested

TABLE 2

| Percent Efficacy of Compounds of Examples 1 to 7 in *In vitro* Biological Evaluation | | | |
|---|---|---|---|
| Example Number | *Erysiphe graminis* | *Puccinia recondita* | Percent Phytotoxicity to Host Plants |
| 1 | 100 | 0 | 0 |
| 2 | 83 | 0 | 0 |
| 3 | 14 | 100 | 0 |
| 4 | 87 | 66 | 0 |
| 5 | 52 | 100 | 0 |
| 6 | 100 | 0 | 0 |
| 7 | 79 | 0 | 0 |
| NT - Not Tested | | | |

**Claims**

**Claims for the following Contracting States : AT, BE, CH, DE, FR, GB, IT, LI, NL, SE**

**1.** A compound of the formula

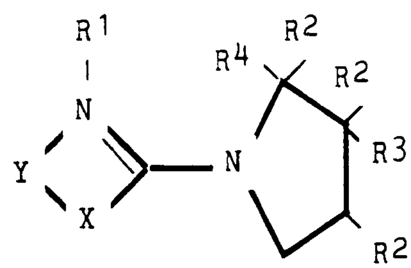

I

or an agriculturally acceptable salt thereof,
wherein:

$R^1$ is hydrogen or $C_1$-$C_3$ alkyl, with the proviso that if the compound is not in the salt form, then $R^1$ is absent;

each $R^2$ is independently hydrogen or $C_1$-$C_4$ alkyl;

$R^3$ is a $C_4$-$C_{20}$ alkyl group, a $C_4$-$C_{20}$ alkenyl group, a phenyl group, a phenyl-$C_1$-$C_3$ alkylene group or a phenyl-$C_2$-$C_3$ alkenylene group wherein the phenyl ring can be substituted with from 1 to 5 halogen atoms, $C_1$-$C_6$ alkyl groups, $C_1$-$C_6$ alkoxy groups, trihalomethyl groups, phenyl groups or phenoxy groups;

$R^4$ is hydrogen or $C_1$-$C_4$ alkyl;

Y is a group of the formula $-(CR^7R^7)_n-$,
wherein n is 2, 3 or 4, each $R^7$ independently is hydrogen or $C_1$-$C_4$ alkyl, $C_1$-$C_4$ hydroxyalkyl, $C_2$-$C_4$ alkoxyalkyl, or a group of the formula $-(CH_2)_q-O(CO)-R^8$,
wherein q is 1, 2 or 3, and $R^8$ is methyl or ethyl; and

X is a group of the formula $-CR^7R^7-$,
wherein each $R^7$ independently is as defined above, or a group of the formula $-N(R^9)-$,
wherein $R^9$ is hydrogen or $C_1$-$C_3$ alkyl.

**2.** A compound as claimed in Claim 1, wherein $R^1$ is hydrogen, methyl, ethyl or is absent.

**3.** A compound as claimed in Claim 1 or 2, wherein Y is a group of the formula $-(CR^7R^7)_3-$, wherein $R^7$ is as defined in Claim 1.

4. A compound as claimed in Claim 3, wherein Y is a group of the formula $-(CH_2)_3-$ or $-CH(CH_3)CH_2CH(CH_3)-$.

5. A compound as claimed in any one of the preceding claims, wherein X is $-CH_2-$ or -NH-.

6. A compound as claimed in any one of the preceding claims, wherein each $R^2$ independently is hydrogen or methyl.

7. A compound as claimed in any one of the preceding claims, wherein $R^4$ is methyl.

8. A compound as claimed in any one of the preceding claims, wherein $R^3$ is a phenyl group, optionally substituted at the 4-position with a $C_3$-$C_6$ alkyl group.

9. The compound as claimed in Claim 1 which is *cis* N-(tetrahydropyrimidin-2-yl)-2-methyl-3-(4-*t*-butylphenyl)pyrrolidinium acetate.

10. The compound as claimed in claim 1 which is *trans* N-(tetrahydropyrimidin-2-yl)-2-methyl-3-(4-*t*-butylphenyl)pyrrolidinium acetate.

11. The compound as claimed in claim 1 which is *cis* N-(tetrahydropyrimidin-2-yl)-2-methyl-3-(4-*t*-butylphenyl)pyrrolidinium chloride.

12. The compound as claimed in claim 1 which is *trans* N-(tetrahydropyrimidin-2-yl)-2-methyl-3-(4-*t*-butylphenyl)pyrrolidinium chloride.

13. The compound as claimed in claim 1 which is *cis* N-(4,6-dimethyltetrahydropyrimidin-2-yl)-2-methyl-3-(4-*t*-butylphenyl)pyrrolidinium chloride.

14. The compound as claimed in claim 1 which is *trans* N-(4,6-dimethyltetrahydropyrimidin-2-yl)-2-methyl-3-(4-*t*-butylphenyl)pyrrolidinium chloride.

15. A fungicidal composition comprising as the active ingredient at least one of the compounds as claimed in any one of the preceding claims in admixture withan inert carrier or diluent.

16. A method for the kill and control of fungal organisms which infest plants which comprises applying to the fungi or to the area where infestation is to be prevented, a fungicidally effective amount of a composition as claimed in Claim 15.

17. A process for preparing a compound of Formula I as defined in Claim 1 which process comprises any one of the following:

(A) reacting a compound of the formula

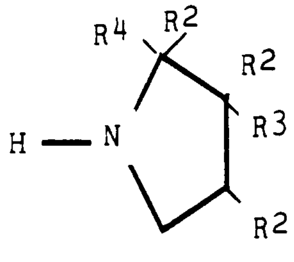

II

wherein $R^2$, $R^3$ and $R^4$ are each as defined in Claim 1,
with a compound of the formula

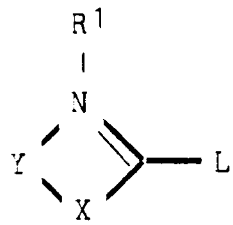

III

wherein $R^1$ is defined in Claim 1, or a halide salt thereof, and L is a suitable leaving group; or
(B) reducing a compound of the formula

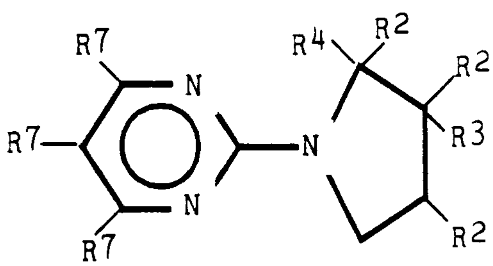

V

wherein:
$R^2$, $R^3$, $R^4$ and $R^7$ are defined as in Claim 1,
using hydrogen and a metallic catalyst to provide the compounds of Formula I where X is -N($R^9$)-.

**18.** A process as claimed in Claim 17(B) wherein the compound of Formula V is prepared by reacting a compound of the formula

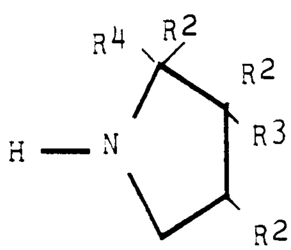

II

wherein:
$R^2$, $R^3$ and $R^4$ are as defined in Claim 1,
with a compound of the formula

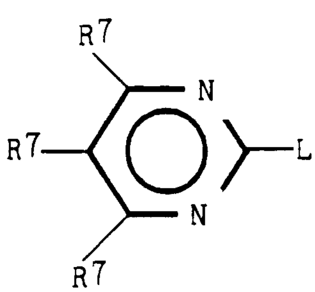

IV

wherein:
$R^7$ is as defined in Claim 1, and L is a suitable leaving group.

**19.** A process as claimed in Claim 18, wherein L is a halogen, and the reaction of the compound of Formula II with the compound of Formula IV is carried out in the presence of a base.

**20.** A process as claimed in any one of Claims 17 to 19, wherein the compound of Formula II is prepared by the internal condensation of a compound of the formula

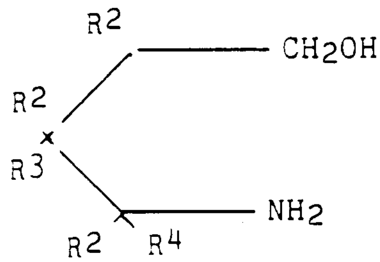

VI

wherein:
$R^2$, $R^3$ and $R^4$ are defined as in Claim 1.

**Claims for the following Contracting State : ES**

**1.** A process for preparing a compound of the formula

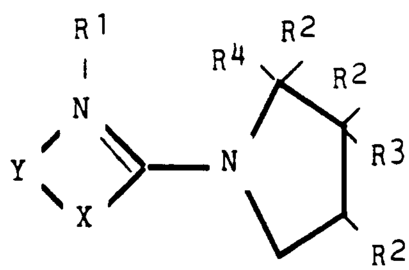

I

or an agriculturally acceptable salt thereof,
wherein:

$R^1$ is hydrogen or $C_1$-$C_3$ alkyl, with the proviso that if the compound is not in the salt form, then $R^1$ is absent;

each $R^2$ is independently hydrogen or $C_1$-$C_4$ alkyl;

$R^3$ is a $C_4$-$C_{20}$ alkyl group, a $C_4$-$C_{20}$ alkenyl group, a phenyl group, a phenyl-$C_1$-$C_3$ alkylene group or a phenyl-$C_2$-$C_3$ alkenylene group wherein the phenyl rings can be substituted with from 1 to 5 halogen atoms, $C_1$-$C_6$ alkyl groups, $C_1$-$C_6$ alkoxy groups, trihalomethyl groups, phenyl groups or phenoxy groups;

$R^4$ is hydrogen or $C_1$-$C_4$ alkyl;

Y is a group of the formula $-(CR^7R^7)_n-$,
wherein n is 2, 3 or 4, each $R^7$ independently is hydrogen or $C_1$-$C_4$ alkyl, $C_1$-$C_4$ hydroxyalkyl, $C_2$-$C_4$ alkoxyalkyl, or a group of the formula $-(CH_2)_q$-O(CO)-$R^8$,
wherein q is 1, 2 or 3, and $R^8$ is methyl or ethyl; and

X is a group of the formula $-CR^7R^7-$,
wherein each $R^7$ independently is as defined above, or a group of the formula $-N(R^9)-$,
wherein $R^9$ is hydrogen or $C_1$-$C_3$ alkyl;

which process comprises any one of the following:

(A) reacting a compound of the formula

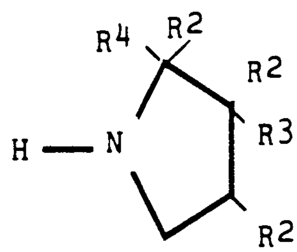

II

wherein $R^2$, $R^3$ and $R^4$ are each as defined as above, with a compound of the formula

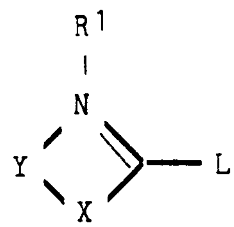

III

wherein $R^1$, Y and X are defined as above, or a halide salt thereof, and L is a suitable leaving group; or

(B) reducing a compound of the formula

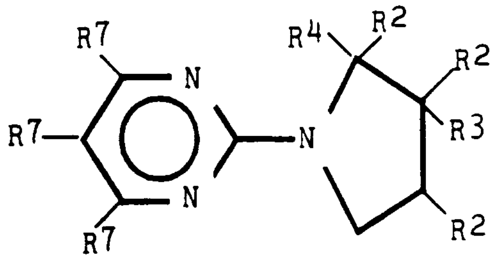

V

wherein:

$R^2$, $R^3$, $R^4$ and $R^7$ are defined as above,

using hydrogen and a metallic catalyst to provide the compounds of Formula I where X is -N($R^9$)-.

**2.** A process as claimed in Claim 1(B) wherein the compound of Formula V is prepared by reacting a compound of the formula

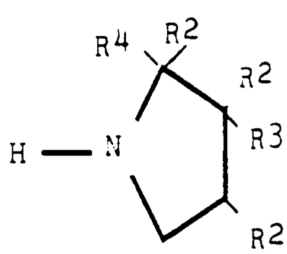

II

wherein:

$R^2$, $R^3$ and $R^4$ are as defined in Claim 1,
with a compound of the formula

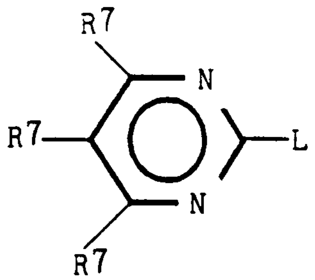

IV

wherein:
$R^7$ is as defined above, and L is a suitable leaving group.

**3.** A process as claimed in Claim 2, wherein L is a halogen, and the reaction of the compound of Formula II with the compound of Formula IV is carried out in the presence of a base.

**4.** A process as claimed in any one of the preceding claims, wherein the compound of Formula II is prepared by the internal condensation of a compound of the formula

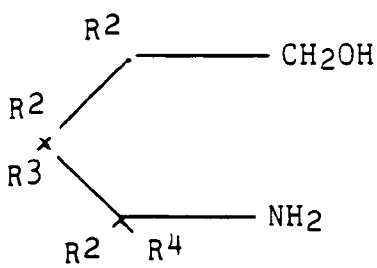

VI

wherein:
$R^2$, $R^3$ and $R^4$ are defined as in Claim 1.

**5.** A process of Claim 1(A) wherein L is an alkoxy, an alkylthio, phenylthio or sulfonic acid ($SO_3H$) group.

**6.** A process of Claim 1(A) or 5 wherein the reaction temperature is from 50 to 200 ° C.

**7.** A process of Claim 6 wherein the reaction is carried out neat or in a suitable organic solvent.

**8.** A process of Claim 7, wherein the suitable organic solvent is ethanol, n-propanol, isopropanol, n-butanol, isobutanol, n-pentanol or n-hexanol.

**9.** A process of Claim 1(B) wherein the metallic catalyst is palladium, platinum, rhodium or ruthenium.

**10.** A process of Claim 1(B) or 9 wherein the reaction is carried out in a polar solvent and in the presence of an acid.

**11.** A process of Claim 10 wherein the polar solvent is water, methanol, ethanol or mixtures thereof and the acid is hydrochloric or acetic acid.

**12.** A process of Claim 2 wherein the reaction is carried out in the presence of a solvent, at a temperature of from 50 to 200 ° C.

**13.** A process of Claim 12 wherein the solvent is ethanol, n-propanol, n-butanol, n-pentanol or n-hexanol, dimethylforamide or dimethylsulfoxide in the presence of triethylamine or ethyldiisopropylamine.

**14.** A process of Claim 4 wherein the reaction is carried out in tetrahydrofuran, at a temperature of from 0 to 150 °C.

**Patentansprüche**

**Patentansprüche für folgende Vertragsstaaten : AT, BE, CH, DE, FR, GB, IT, LI, NL, SE**

**1.** Verbindung der Formel

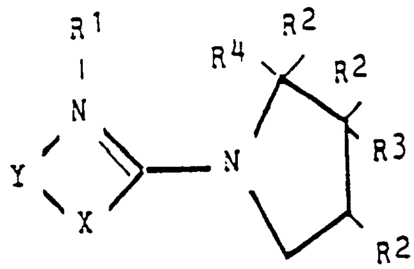

I

oder ein landwirtschaftlich verträgliches Salz davon,
worin:

R1 Wasserstoff oder $C_1$-$C_3$-Alkyl ist, mit der Maßgabe, daß, wenn die Verbindung nicht als Salz vorliegt, $R^1$ fehlt,

$R^2$ jeweils unabhängig Wasserstoff oder $C_1$-$C_4$-Alkyl ist,

$R^3$ eine $C_4$-$C_{20}$-Alkyl-Gruppe, eine $C_4$-$C_{20}$ Alkenyl-Gruppe, eine Phenyl-Gruppe, eine Phenyl-$C_1$-$C_3$-Alkenyl-Gruppe oder eine Phenyl-$C_2$-$C_3$-Alkenylen-Gruppe ist, in der der Phenyl-Ring mit 1 bis 5 Halogenatomen, $C_1$-$C_6$-Alkyl-Gruppen, $C_1$-$C_6$-Alkoxy-Gruppen, Trihalogenmethyl-Gruppen, Phenyl-Gruppen oder Phenoxy-Gruppen substituiert sein kann,

$R^4$ Wasserstoff oder $C_1$-$C_4$-Alkyl ist,

Y eine Gruppe der Formel $-(CR^7R^7)_n-$ ist, worin n 2, 3 oder 4 ist, $R^7$ jeweils unabhängig Wasserstoff oder $C_1$-$C_4$-Alkyl, $C_1$-$C_4$-Hydroxyalkyl, $C_2$-$C_4$ Alkoxyalkyl oder eine Gruppe der Formel $-(CH_2)_q-O-(CO)-R^8$ ist, worin q 1, 2 oder 3 ist und $R^8$ Methyl oder Ethyl ist, und

X eine Gruppe der Formel $-CR^7R^7-$ ist, worin $R^7$ jeweils unabhängig wie vorstehend definiert ist oder eine Gruppe der Formel $-N(R^9)-$ ist, worin $R^9$ Wasserstoff oder $C_1$-$C_3$-Alkyl ist.

**2.** Verbindung nach Anspruch 1, worin $R^1$ Wasserstoff, Methyl oder Ethyl ist, oder fehlt.

**3.** Verbindung nach Anspruch 1 oder 2, worin Y eine Gruppe der Formel $-(CR^7R^7)_3-$ ist, worin $R^7$ wie in Anspruch 1 definiert ist.

**4.** Verbindung nach Anspruch 3, worin Y eine Gruppe der Formel $-(CH_2)_3-$ oder $-CH(CH_3)CH_2CH(CH_3)-$ ist.

**5.** Verbindung nach einem der vorhergehenden Ansprüche, worin X $-CH_2-$ oder -NH- ist.

**6.** Verbindung nach einem der vorhergehenden Ansprüche, worin $R^2$ jeweils unabhängig Wasserstoff oder Methyl ist.

**7.** Verbindung nach einem der vorhergehenden Ansprüche, worin $R^4$ Methyl ist.

**8.** Verbindung nach einem der vorhergehenden Ansprüche, worin $R^3$ eine Phenyl-Gruppe ist, die wahlweise an Position 4 mit einer $C_3$-$C_6$ Alkyl-Gruppe substituiert ist.

**9.** Verbindung nach Anspruch 1, die cis-N-(Tetrahydropyrimidin-2-yl)-2-methyl-3-(4-t-butylphenyl)-pyrrolidiniumacetat ist.

**10.** Verbindung nach Anspruch 1, die trans-N-(Tetrahydropyrimidin-2-yl)-2-methyl-3-(4-t-butylphenyl)-pyrrolidiniumacetat ist.

**11.** Verbindung nach Anspruch 1, die cis-N-(Tetrahydropyrimidin-2-yl)-2-methyl-3-(4-t-butylphenyl)-pyrrolidiniumchlorid ist.

**12.** Verbindung nach Anspruch 1, die trans-N-(Tetrahydropyrimidin-2-yl)-2-methyl-3-(4-t-butylphenyl)-pyrrolidiniumchlorid ist.

**13.** Verbindung nach Anspruch 1, die cis-N-(4,6-Dimethyltetrahydropyrimidin-2-yl)-2-methyl-3-(4-t-butylphenyl)pyrrolidiniumchlorid ist.

**14.** Verbindung nach Anspruch 1, die trans-N-(4,6-Dimethyltetrahydropyrimidin-2-yl)-2-methyl-3-(4-t-butylphenyl)pyrrolidiniumchlorid ist.

**15.** Fungizide Verbindung, die als Wirkstoff mindestens eine der Verbindungen nach den vorhergehenden Ansprüchen in einem Gemisch mit einem inerten Träger oder Verdünnungsmittel enthält.

**16.** Verfahren zum Abtöten und Kontrollieren von Pflanzen befallenden Pilz-Organismen, die Aufbringen einer fungizid wirksamen Menge einer Zusammensetzung nach Anspruch 15 auf den Pilz oder das Gebiet in dem Befall verhindert werden soll, umfaßt.

**17.** Verfahren zur Herstellung einer Verbindung der Formel I, wie in Anspruch 1 definiert, das einen der folgenden Schritte umfaßt:

(A) Umsetzten der Verbindung der Formel

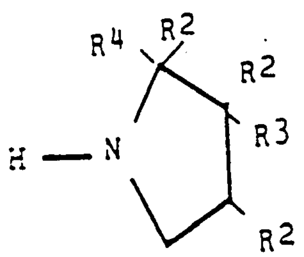

II

worin $R^2$, $R^3$ und $R^4$ jeweils wie in Anspruch 1 definiert sind, mit einer Verbindung der Formel

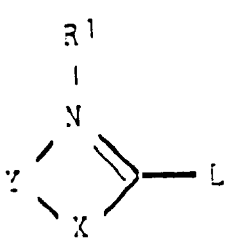

III

worin $R^1$ wie in Anspruch 1 definiert, oder einem Halogenid-Salz davon, und L eine geeignete Austrittsgruppe ist, oder

(B) Reduzieren einer Verbindung der Formel

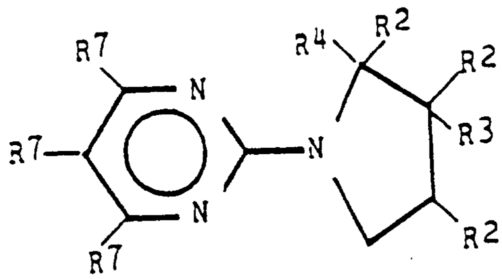

V

worin:

$R^2$, $R^3$, $R^4$ und $R^7$ wie in Anspruch 1 definiert sind,

unter Verwendung von Wasserstoff und einem metallischen Katalysator, wobei Verbindungen der Formel I, in denen X - N(R9)- ist, geliefert werden.

**18.** Verfahren nach Anspruch 17(B), wobei die Verbindung der Formel V durch Umsetzten einer Verbindung der Formel

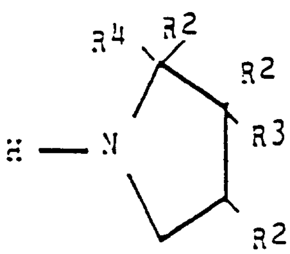

II

worin:

$R^2$, $R^3$ und $R^4$ wie in Anspruch 1 definiert sind,

mit einer Verbindung der Formel

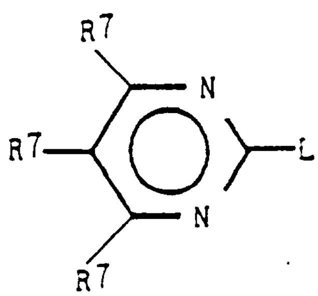

IV

worin:

R7 wie in Anspruch 1 definiert ist, und L eine geeignete Austrittsgruppe ist.

**19.** Verfahren nach Anspruch 18, wobei L ein Halogen ist, und die Reaktion der Verbindung der Formel II mit der Verbindung der Formel IV in Anwesenheit einer Base durchgeführt wird.

**20.** Verfahren nach einem der Ansprüche 17 bis 19, wobei die Verbindung der Formel II durch die interne Kondensation einer Verbindung der Formel

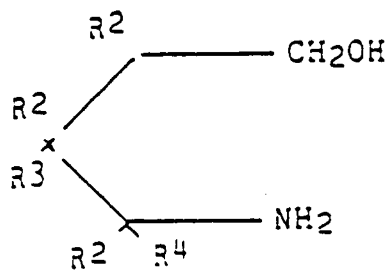

VI

worin:

$R^2$,$R^3$ und $R^4$ wie in Anspruch 1 definiert sind, hergestellt wird.

**Patentansprüche für folgenden Vertragsstaat : ES**

**1.** Verfahren zu Herstellung einer Verbindung der Formel

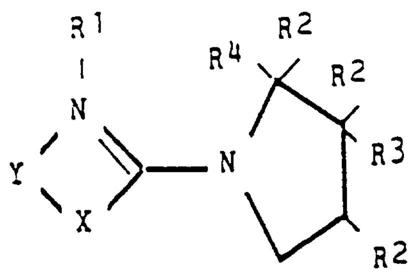

I

oder einem landwirtschaftlich verträglichen Salz davon, worin:

$R^1$ Wasserstoff oder $C_1$-$C_3$-Alkyl ist, mit der Maßgabe, daß, wenn die Verbindung nicht als Salz vorliegt, $R^1$ fehlt,

$R^2$ jeweils unabhängig Wasserstoff oder $C_1$-$C_4$-Alkyl ist,

$R^3$ eine $C_4$-$C_{20}$-Alkyl-Gruppe, eine $C_4$-$C_{20}$ Alkenyl-Gruppe, eine Phenyl-Gruppe, eine Phenyl-$C_1$-$C_3$-Alkenyl-Gruppe oder eine Phenyl-$C_2$-$C_3$-Alkenylen-Gruppe ist, in der der Phenyl-Ring mit 1 bis 5 Halogenatomen, $C_1$-$C_6$-Alkyl-Gruppen, $C_1$-$C_6$-Alkoxy-Gruppen, Trihalogenmethyl-Gruppen, Phenyl-Gruppen oder Phenoxy-Gruppen substituiert sein kann,

$R^4$ Wasserstoff oder $C_1$-$C_4$-Alkyl ist,

Y eine Gruppe der Formel $-(CR^7R^7)_n-$ ist, worin n 2, 3 oder 4 ist, $R^7$ jeweils unabhängig Wasserstoff oder $C_1$-$C_4$-Alkyl, $C_1$-$C_4$-Hydroxyalkyl, $C_2$-$C_4$-Alkoxyalkyl oder eine Gruppe der Formel $-(CH_2)_q$-O-(CO)-$R^8$ ist, worin q 1, 2 oder 3 ist und $R^8$ Methyl oder Ethyl ist, und

X eine Gruppe der Formel $-CR^7R^7-$ ist, worin $R^7$ jeweils unabhängig wie vorstehend definiert ist oder eine Gruppe der Formel $-N(R^9)-$ ist, worin $R^9$ Wasserstoff oder $C_1$-$C_3$-Alkyl ist, wobei das Verfahren einen der folgenden Schritte umfaßt:

(A) Umsetzten der Verbindung der Formel

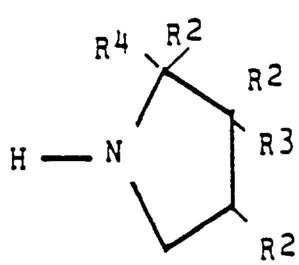

II

worin $R^2$, $R^3$ und $R^4$ jeweils wie vorstehend definiert sind, mit einer Verbindung der Formel

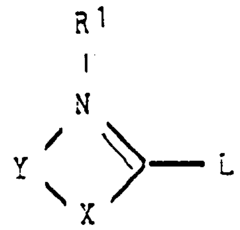

III

worin $R^1$, Y und X wie vorstehend definiert ist, oder einem Halogen-Salz davon, und L eine geeignete Austritts-Gruppe ist, oder
(B) Reduzieren einer Verbindung der Formel

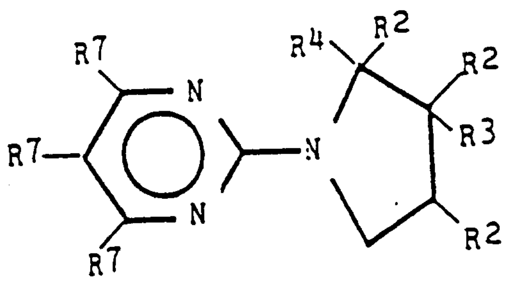

V

worin:
$R^2$, $R^3$, $R^4$ und $R^7$ wie vorstehend definiert sind,
unter Verwendung von Wasserstoff und einem metallischen Katalysator, wobei Verbindungen der Formel I, in denen X -N($R^9$)- ist, geliefert werden.

**2.** Verfahren nach Anspruch 1(B), wobei die Verbindung der Formel V durch Umsetzten einer Verbindung der Formel

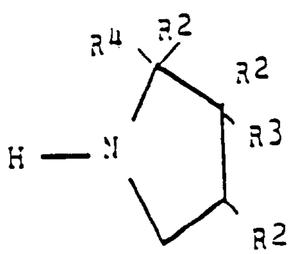

II

worin:
$R^2$, $R^3$ und $R^4$ wie in Anspruch 1 definiert sind, mit einer Verbindung der Formel

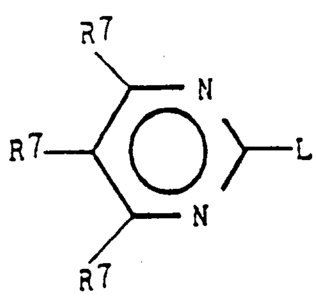

IV

worin:
$R^7$ wie vorstehend definiert ist, und L eine geeignete Autritts-Gruppe ist.

**3.** Verfahren nach Anspruch 2, wobei L ein Halogen ist, und die Reaktion der Verbindung der Formel II mit der Verbindung der Formel IV in Anwesenheit einer Base durchgeführt wird.

**4.** Verfahren nach einem der vorhergehenden Ansprüche, wobei die Verbindung der Formel II durch die interne Kondensation einer Verbindung der Formel

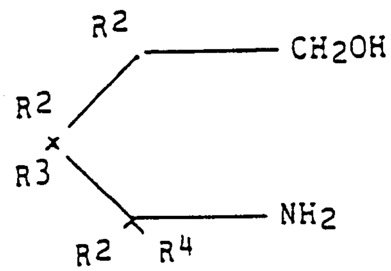

VI

worin:
$R^2$,$R^3$ und $R^4$ wie in Anspruch 1 definiert sind, hergestellt wird.

**5.** Verfahren nach Anspruch 1(A), wobei L eine Alkoxy-, eine Alkylthio-, eine Phenylthio- oder eine Sulfonsäure- ($SO_3H$) Gruppe ist.

**6.** Verfahren nach Anspruch 1(A) oder 5, wobei die Reaktionstemperatur 50 bis 200 °C ist.

**7.** Verfahren nach Anspruch 6, wobei die Reaktion unverdünnt oder in einem geeigneten organischen Lösungsmittel durchgeführt wird.

**8.** Verfahren nach Anspruch 7, wobei das geeignete organische Lösungsmittel Ethanol, n-Propanol, Isopropanol, n-Butanol, Isobutanol, n-Pentanol oder n-Hexanol ist.

**9.** Verfahren nach Anspruch 1(B), wobei der metallische Katalysator Palladium, Platin, Rhodium oder Ruthenium ist.

**10.** Verfahren nach Anspruch 1(B) oder 9, wobei die Reaktion in einem polaren Lösungsmittel und in Anwesenheit einer Säure durchgeführt wird.

**11.** Verfahren nach Anspruch 10, wobei das polare Lösungsmittel Wasser, Methanol, Ethanol oder Gemische davon sind, und die Säure Chlorwasserstoffsäure oder Essigsäure ist.

**12.** Verfahren nach Anspruch 2, wobei die Reaktion in Anwesenheit eines Lösungsmittels bei einer Temperatur von 50° bis 200 °C durchgeführt wird.

**13.** Verfahren nach Anspruch 12, wobei das Lösungsmittel Ethanol, n-Propanol, n-Butanol, n-Pentanol oder n-Hexanol, Dimethylformamid oder Dimethylsulfoxid in Anwesenheit von Triethylamin oder Ethyldiisopropylamin ist.

**14.** Verfahren nach Anspruch 4, wobei die Reaktion in Tetrahydrofuran bei einer Temperatur von 0° bis 150 °C durchgeführt wird.

## Revendications

### Revendications pour les Etats contractants suivants : AT, BE, CH, DE, FR, GB, IT, LI, NL, SE

**1.** Composé de formule

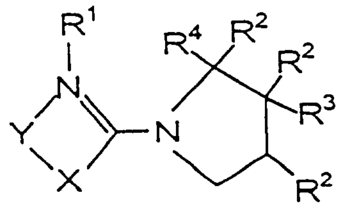

(I)

ou un de ses sels compatible avec une utilisation en agriculture, dans laquelle :

$R^1$ représente un atome d'hydrogène ou un groupe alkyle en $C_1$-$C_3$, à condition que $R^1$ soit absent au cas où le composé n'est pas sous forme de sel;

chaque $R^2$ représente indépendamment un atome d'hydrogène ou un groupe alkyle en $C_1$-$C_4$;

$R^3$ représente un groupe alkyle en $C_4$-$C_{20}$, alcényle en $C_4$-$C_{20}$, phényle, phényl-alcoylène en $C_1$-$C_3$ ou un groupe phényl-alcoylidène en $C_2$-$C_3$, dans lequel le noyau phényle peut être substitué par 1 à 5 atomes d'halogène, groupes alkyle en $C_1$-$C_6$, alkoxy en $C_1$-$C_6$, trihalométhyle, phényle ou phénoxy;

$R^4$ représente un atome d'hydrogène ou un groupe alkyle en $C_1$-$C_4$;

Y représente un groupe de formule $-(CR^7R^7)_n-$, dans laquelle n est égal à 2,3 ou 4, chaque $R^7$ représentant indépendamment un atome d'hydrogène ou un groupe alkyle en $C_1$-$C_4$, hydroxyalkyle en $C_1$-$C_4$, alkoxyalkyle en $C_2$-$C_4$ ou un groupe de formule $-(CH_2)_q-O(CO)-R^8$, dans laquelle q est égal à 1,2 ou 3, et $R^8$ est un groupe méthyle ou éthyle; et

X représente un groupe de formule $-CR^7R^7-$, dans laquelle chaque $R^7$ représente indépendamment les mêmes termes que ceux définis ci-dessus, ou un groupe de formule $-N(R^9)-$, dans laquelle $R^9$ est un atome d'hydrogène ou un groupe alkyle en $C_1$-$C_3$.

**2.** Composé conforme à la revendication 1, dans lequel $R^1$ est absent ou représente un atome d'hydrogène, un groupe méthyle ou éthyle.

**3.** Composé conforme aux revendications 1 ou 2, dans lequel Y est un groupe de formule $-(CR^7R^7)_3-$, dans laquelle $R^7$ a la même signification que celle définie dans la revendication 1.

**4.** Composé conforme à la revendication 3, dans lequel Y est un groupe de formule $-(CH_2)_3-$ ou $-CH(CH_3)CH_2CH(CH_3)-$.

**5.** Composé conforme à une des revendications précédentes, dans lequel X représente un groupe $-CH_2-$ ou -NH-.

**6.** Composé conforme à une des revendications précédentes, dans lequel chaque $R^2$ représente indépendamment un atome d'hydrogène ou un groupe méthyle.

**7.** Composé conforme à une des revendications précédentes, dans lequel $R^4$ représente un groupe méthyle.

**8.** Composé conforme à une des revendications précédentes, dans lequel $R^3$ représente un groupe phényle, éventuellement substitué en position 4 par un groupe alkyle en $C_3$-$C_6$.

**9.** Le composé conforme à la revendication 1 étant l'acétate de *cis* N-(tétrahydropyrimidin-2-yl)-2-méthyl-3-(4-*t*-butylphényl)pyrrolidinium.

**10.** Le composé conforme à la revendication 1 étant l'acétate de*trans* N-(tétrahydropyrimidin-2-yl)-2-méthyl-3-(4-*t*-butylphényl)pyrrolidinium.

**11.** Le composé conforme à la revendication 1 étant le chlorure de*cis* N-(tétrahydropyrimidin-2-yl)-2-méthyl-3-(4-*t*-butylphényl)pyrrolidinium.

**12.** Le composé conforme à la revendication 1 étant le chlorure de *trans* N-(tétrahydropyrimidin-2-yl)-2-méthyl-3-(4-*t*-butylphényl)pyrrolidinium.

**13.** Le composé conforme à la revendication 1 étant le chlorure de *cis* N-(4,6-diméthyltétrahydropyrimidin-2-yl)-2-méthyl-3-(4-*t*-butylphényl)pyrrolidinium.

**14.** Le composé conforme à la revendication 1 étant le chlorure de *trans* N-(4,6-diméthyltétrahydropyrimidin-2-yl)-2-méthyl-3-(4-*t*-butylphényl)pyrrolidinium.

**15.** Composition fongicide comprenant comme ingrédient actif au moins un des composés conformes à une des revendications précédentes, mélangé avec un diluant ou un support inerte.

**16.** Méthode de destruction et de lutte contre des organismes fongiques infestant les plantes, cette méthode comprenant l'application de la composition conforme à la renvendication 15, en quantité suffisante pour avoir un effet fongicide, sur un champignon ou une zone à protéger contre l'infestation.

**17.** Procédé pour la préparation d'un composé de formule I conforme à la revendication 1, ce procédé comprenant une des réactions suivantes :
(A) réaction d'un composé de formule

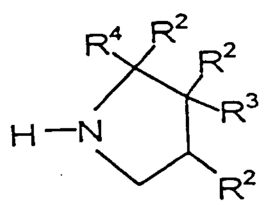

(II)

dans laquelle $R^2$, $R^3$ et $R^4$ ont chacun la signification définie dans la revendication 1,
avec un composé de formule

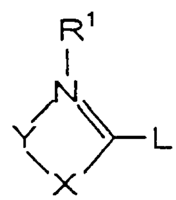

(III)

dans laquelle $R^1$ a la même signification que dans la revendication 1 et L est un groupe partant approprié, ou avec un halogénure d'un tel composé; ou
(B) réduction d'un composé de formule

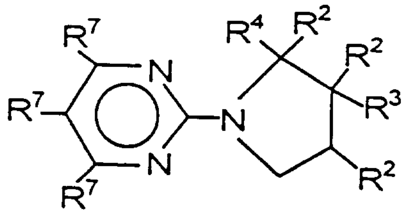

(V)

dans laquelle $R^2$, $R^3$, $R^4$ et $R^7$ sont définis comme dans la revendication 1,
en utilisant de l'hydrogène et un catalyseur métallique pour obtenir des composés de formule I dans laquelle X est un groupe -N($R^9$)-.

**18.** Procédé conforme à la revendication 17 (B), dans lequel le composé de formule V est préparé par réaction d'un composé de formule

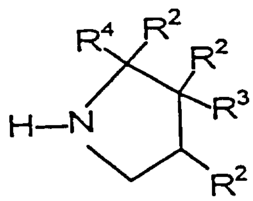

(II)

dans laquelle $R^2$, $R^3$, et $R^4$ sont ceux définis dans la revendication 1, avec un composé de formule

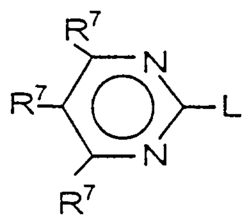

(IV)

dans laquelle la signification de $R^7$ est celle définie dans la revendication 1, et dans laquelle L représente un groupe partant approprié.

**19.** Procédé conforme à la revendication 18, dans lequel L est un atome d'halogène, et dans lequel la réaction du composé de formule (II) avec le composé de formule (IV) est réalisée en présence d'une base.

**20.** Procédé conforme à une des revendications 17 à 19, dans lequel le composé de formule (II) est préparé par condensation interne d'un composé de formule

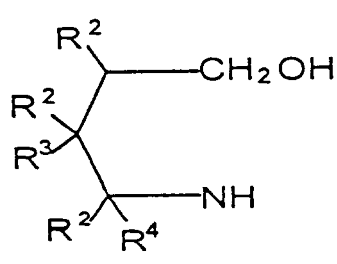

(VI)

dans laquelle $R^2$, $R^3$ et $R^4$ sont ceux définis dans la revendication 1.

**Revendications pour l'Etat contractant suivant : ES**

**1.** Procédé de préparation d'un composé de formule

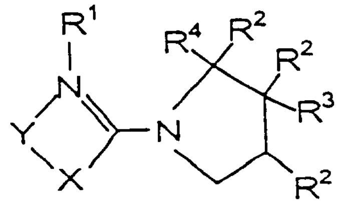

(I)

ou un de ses sels compatible avec une utilisation en agriculture, dans laquelle :

$R^1$ représente un atome d'hydrogène ou un groupe alkyle en $C_1$-$C_3$, à condition que $R^1$ soit absent au cas où le composé n'est pas sous forme de sel,

chaque $R^2$ représente indépendamment un atome d'hydrogène ou un groupe alkyle en $C_1$-$C_4$;

$R^3$ représente un groupe alkyle en $C_4$-$C_{20}$, alcényle en $C_4$-$C_{20}$, phényle, phényl-alcoylène en $C_1$-$C_3$ ou un groupe phényl-alcoylidène en $C_2$-$C_3$, dans lequel le groupe phényle peut être substitué par 1 à 5 atomes d'halogène, groupes alkyle en $C_1$-$C_6$, alkoxy en $C_1$-$C_6$, trihalométhyle, phényle ou phénoxy;

$R^4$ représente un atome d'hydrogène ou un groupe alkyle en $C_1$-$C_4$;

Y représente un groupe de formule $-(CR^7R^7)_n-$, dans laquelle n est égal à 2,3 ou 4, chaque $R^7$ représentant indépendamment un atome d'hydrogène ou un groupe alkyle en $C_1$-$C_4$, hydroxyalkyle en $C_1$-$C_4$, alkoxyalkyle en $C_2$-$C_4$ ou un groupe de formule $-(CH_2)_q-O(CO)-R^8$, dans laquelle q est égal à 1,2 ou 3, et $R^8$ est un groupe méthyle ou éthyle; et

X représente un groupe de formule $-CR^7R^7-$, dans laquelle chaque $R^7$ représente indépendamment les mêmes termes que ceux définis ci dessus, ou un groupe de formule $-N(R^9)-$, dans laquelle $R^9$ est un atome d'hydrogène ou un groupe alkyle en $C_1$-$C_3$;

ce procédé comprenant une des réactions suivantes :

(A) réaction d'un composé de formule

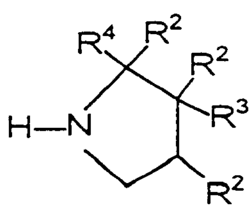

(II)

dans laquelle $R^2$, $R^3$ et $R^4$ ont chacun la signification définie ci-dessus,

avec un composé de formule

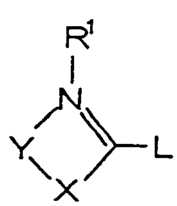

(III)

dans laquelle $R^1$ , Y et X ont la signification définie ci-dessus et L est un groupe partant approprié, ou avec un halogénure d'un tel composé; ou

(B) réduction d'un composé de formule

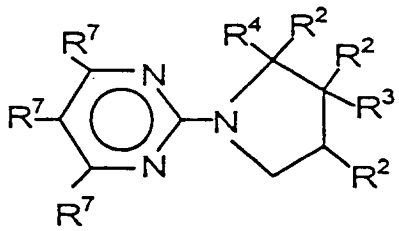

(V)

dans laquelle $R^2$, $R^3$, $R^4$ et $R^7$ sont définis comme ci-dessus,
en utilisant de l'hydrogène et un catalyseur métallique pour obtenir des composés de formule I dans laquelle X est un groupe -N($R^9$)-.

**2.** Procédé conforme à la revendication 1(B), dans lequel le composé de formule V est préparé par réaction d'un composé de formule

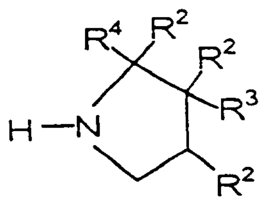

(II)

dans laquelle $R^2$, $R^3$, et $R^4$ sont ceux définis dans la revendication 1,
avec un composé de formule

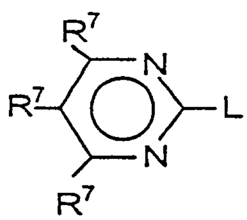

(IV)

dans laquelle $R^7$ a la signification définie ci-dessus.

**3.** Procédé conforme à la revendication 2, dans lequel L est un atome d'halogène, et la réaction du composé de formule (II) avec le composé de fromule (IV) est réalisée en présence d'une base.

**4.** Procédé conforme à une des revendications précédentes, dans lequel le composé de formule (II) est préparé par condensation interne d'un composé de formule

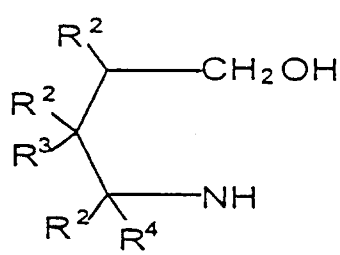

(VI)

dans laquelle $R^2$, $R^3$, et $R^4$ sont définis comme dans la revendication 1.

**5.** Procédé conforme à la revendication 1(A), dans lequel L est un groupe alkoxy, alkylthio, phénylthio ou d'acide sulfonique ($SO_3H$).

**6.** Procédé conforme à la revendication 1(A) ou 5, dans lequel la température de réaction est comprise entre 50 et 200 °C.

**7.** Procédé conforme à la revendication 6, dans lequel la réaction est réalisée en milieu non dilué ou dans un solvant organique approprié.

**8.** Procédé conforme à la revendication 7, dans lequel le solvant organique approprié est l'éthanol, le n-propanol, l'isopropanol, le n-butanol, l'isobutanol, le pentanol ou le n-hexanol.

**9.** Procédé conforme à la revendication 1(B), dans lequel le catalyseur métallique est le palladium, le platine, le rhodium ou le ruthénium.

**10.** Procédé conforme à la revendication 1(B) ou 9, dans lequel la réaction est réalisée dans un solvant polaire et en présence d'un acide.

**11.** Procédé conforme à la revendication 10, dans lequel le solvant polaire est l'eau, le méthanol, l'éthanol ou un mélange de ceux-ci, et l'acide est l'acide chlorhydrique ou l'acide acétique.

**12.** Procédé conforme à la revendication 2, dans lequel la réaction est réalisée en présence d'un solvant, à une température comprise entre 50 et 200 °C.

**13.** Procédé conforme à la revendication 12, dans lequel le solvant est l'éthanol, le n-propanol, le n-butanol, le n-pentanol ou le n-hexanol, le diméthylformamide ou le diméthylsulfoxyde en présence de triétylamine ou d'éthyldiisoproylamine.

**14.** Procédé conforme à la revendication 4, dans lequel la réaction est réalisée dans le tétrahydrofurane à une température comprise entre 0 et 150 °C.